## Europäisches Patentamt
## European Patent Office
## Office européen des brevets

(19)

(11) Veröffentlichungsnummer: **0 031 149**
**B1**

(12)

# EUROPÄISCHE PATENTSCHRIFT

(45) Veröffentlichungstag der Patentschrift:
15.02.84

(51) Int. Cl.³: **G 01 N 27/46,** G 01 N 33/96,
A 61 B 5/14

(21) Anmeldenummer: 80108028.4

(22) Anmeldetag: 18.12.80

(54) Verfahren zum Kalibrieren einer Messanordnung zur Bestimmung des Sauerstoffpartialdrucks in Blut.

(30) Priorität: 20.12.79 DE 2951325

(43) Veröffentlichungstag der Anmeldung:
01.07.81 Patentblatt 81/26

(45) Bekanntmachung des Hinweises auf die Patenterteilung:
15.02.84 Patentblatt 84/7

(84) Benannte Vertragsstaaten:
AT BE CH DE FR GB IT LI LU NL SE

(56) Entgegenhaltungen:
DE - A - 2 141 172
DE - A - 2 414 365
DE - A - 2 543 543
DE - A - 2 621 677
DE - A - 2 645 736

(73) Patentinhaber: Hellige GmbH,
Postfach 728 Heinrich-von-Stephan-Strasse 4,
D-7800 Freiburg (DE)

(72) Erfinder: Hopmeier, Joachim, Dipl.-Ing., Pfädle 8,
D-7800 Freiburg (DE)
Erfinder: Leist, Helmut, Dipl.-Ing., Waldallee 34,
D-7800 Freiburg (DE)
Erfinder: Ullrich, Georg J., Dipl.-Phys., Mettackerweg 84,
D-7800 Freiburg (DE)

(74) Vertreter: Patentanwälte TER MEER - MÜLLER -
STEINMEISTER, Triftstrasse 4, D-8000 München 22 (DE)

ACTORUM AG

Verfahren zum Kalibrieren einer Messanordnung zur Bestimmung des Sauerstoffpartialdrucks in Blut

Die Erfindung betrifft ein Verfahren gemäss dem Oberbegriff des Anspruchs 1.

Die Erfindung bezieht sich ausserdem auf eine Anwendung des Verfahrens sowie auf ein Kalibriermittel zur Herstellung einer bei dem Verfahren verwendeten Null-Kalibrierlösung und eine Null-Kalibrierlösung zur Verwendung in dem Verfahren.

Wegen der Bedeutung für die transcutane Bestimmung des Partialdrucks von Sauerstoff im Blut wird das erfindungsgemässe Verfahren für diese Anwendungsart näher beschrieben.

Es ist bekannt, elektrochemische Messaufnehmer für Sauerstoffpartialdrücke dadurch zu kalibrieren, dass sie zwei bekannten Sauerstoffpartialdrücken ausgesetzt werden, zum Beispiel Luft mit ihrem aus dem barometrischen Druck abzuleitenden bekannten Sauerstoffpartialdruck und reinem Stickstoff mit dem Sauerstoffpartialdruck Null. Die Verwendung reinen Stickstoffs mit der Notwendigkeit der Bereithaltung unhandlicher Gasflaschen ist aber oft sehr unbequem. Daher werden anstelle des Stickstoffs oder eines anderen sauerstofffreien Gases auch Lösungen von sauerstoffbindenden Substanzen in Wasser — sogenannte Null-Kalibrierlösungen — benutzt, um an der Messfläche des Messaufnehmers den Sauerstoffpartialdruck Null herzustellen. Zu diesem Zweck werden der Messaufnehmer entweder in die Lösung getaucht oder die Messfläche des Messaufnehmers wird so mit der Lösung bedeckt, dass kein Sauerstoffzutritt möglich ist. Ein Kalibrierverfahren dieser Art, insbesondere für Messaufnehmer zur transcutanen Messung des Sauerstoffpartialdrucks im Blut, ist in DE-A-2 645 736 beschrieben.

Die Benutzung der Null-Kalibrierlösung hat jedoch den Nachteil, dass diese allmählich unwirksam wird. Durch Luftzutritt während des Öffnens des Behälters oder Fläschchens, in dem die Lösung aufbewahrt wird, beim Gebrauch, sowie durch eventuell versehentliches längeres Offenstehenlassen und auch durch fortwährende Sauerstoffdiffusion, insbesondere durch die Wände von Kunststoffbehältern, wird allmählich das Sauerstoffbindevermögen des Bindungsmittels — zum Beispiel Natriumsulfit $Na_2SO_3$ oder Natriumdithionit $Na_2S_2O_4$, erschöpft. Spätestens, wenn das gesamte Natriumsulfit durch Sauerstoffbindung in Natriumsulfat verwandelt ist, ist dieses Bindungsmittel unwirksam und die Null-Kalibrierlösung unbrauchbar.

Um sicher zu gehen, dass beim Kalibriervorgang eine brauchbare Null-Kalibrierlösung benutzt wird, wird daher auch vielfach empfohlen, diese stets frisch anzusetzen. Dies ermöglicht zwar eine Aussage über die Funktionstüchtigkeit des Messaufnehmers bei der Kalibrierung, ist aber umständlich, zeitraubend und teuer.

Insbesondere beim Einsatz von Sauerstoffmessgeräten in der Medizin, z.B. solchen, die fortlaufend den Partialdruck des Sauerstoffs im Blut messen, ist es im Interesse der Sicherheit für den Patienten erforderlich, jederzeit ohne umständliche Vorarbeiten und ohne ständige Bereithaltung an sich überflüssiger Gegenstände, wie Stickstoff-Gasflaschen, in der Umgebung des Bettes, aber sicher und verlässlich die einwandfreie Funktion des Sauerstoff-Messgeräts und damit seinen Kalibrierzustand, speziell die richtige Lage seines Nullpunktes, überprüfen zu können.

Dies sicherzustellen, war die Aufgabe, die durch das Verfahren gemäss Anspruch 1 in überraschend einfacher Weise gelöst wurde.

Eine Anwendungsmöglichkeit des Verfahrens ist in Anspruch 4 definiert. Ein erfindungsgemässes Kalibriermittel zur Herstellung einer Null-Kalibrierlösung sowie eine erfindungsgemässe Null-Kalibrierlösung sind Gegenstand der Ansprüche 5 bzw. 6.

Bei der Erfindung wurde von dem Umstand ausgegangen, dass mit dem Verbrauch der Null-Kalibrierlösung, das heisst mit der allmählichen Umsetzung von z.B. Natriumsulfit in Natriumsulfat, eine Verschiebung des pH-Wertes der Lösung einhergeht. Es hat sich gezeigt, dass es möglich ist, den pH-Wert zu definieren, bei dem die Sauerstoffbindefähigkeit so weit abgenommen hat, dass die Lösung als «unbrauchbar» zu bezeichnen ist. Beim genannten Beispiel der Lösung von Natriumsulfit bewegt sich zum Beispiel der pH-Wert von pH = 9,1 bei guter Bindefähigkeit zu pH = 8,4 bei gerade noch brauchbarer Sauerstoffbindefähigkeit. Somit kann durch Beifügung eines geeigneten, auf den pH-Wert ansprechenden Farbindikators zur Null-Kalibrierlösung bzw. durch Verwendung einer Mischung aus dem Bindungsmittel und dem Farbindikator zur Herstellung der Null-Kalibrierlösung deren Brauchbarkeit durch Farbänderung angezeigt werden. Für das vorgenannte Beispiel hat sich z.B. Thymolblau als Indikator als geeignet erwiesen, dessen Farbe sich vom Blau nach Gelb hin ändert, bevor die Lösung unbrauchbar geworden ist. Thymolblau ist der gebräuchliche Name für die chemische Verbindung Thymolsulfonphthalein ($C_{27}H_{30}O_5S$).

Bei der Bestimmung des Partialdrucks von Sauerstoff im Blut können die Messaufnehmer sowohl für transcutane als auch für intravasale oder subcutane Applikation eingerichtet sein. Für das Kalibrierverfahren ergeben sich aufgrund der Anwendung keine wesentlichen Unterschiede.

Aus der DE-A-2 543 543 ist es bereits bekannt, den Oxidationsgrad eines Speisefettes dadurch zu bestimmen, dass man eine Probe des Fettes nimmt, diese in alkoholischer Kalilauge löst und dann mit einem auf den pH-Wert ansprechenden Farbindikator wie z.B. Thymolblau versetzt und die Farbänderung beobachtet.

Das für das Verfahren gemäss der Anmeldung dienende Eichmittel kann aus einer Mischung des Bindungsmittels und des Farbindikators in geeigneten Mengenverhältnissen in Form von Pulver, Granulat oder Tabletten bestehen und für die Herstellung der Null-Kalibrierlösung vor jeder Messung oder auf Vorrat für mehrere hintereinander ablaufende Messungen bereitgehalten werden. Für Sauerstoff als gesuchter Stoff wird zur Herstellung des Eichmittels Natriumsulfit oder Natriumdithionit in einer Menge von 5 bis 15, insbesondere von ca. 10 g mit einer Menge von Thymolblau zwischen 0,5 bis 10 mg, insbesondere 1 mg gemischt. Zur Herstellung der Kalibrierlösung verwendet man für diese Menge des Eichmittels etwa 300 ml Wasser, möglichst destilliertes Wasser. Es ist zweckmässig, den zur Aufbewahrung der Null-Kalibrierlösung dienenden Behälter, z.B. ein Fläschchen, mit einer Farbkennzeichnung zu versehen, um zu erkennen, wie weit die Kalibrierlösung noch wirksam und brauchbar ist. Entweder werden die beiden wesentlichen Zustände «brauchbar» und «nicht mehr brauchbar» bzw. «verbraucht» durch zwei Farbschilder gekennzeichnet, welche die beiden betreffenden Farben der Kalibrierlösung anzeigen. Oder es genügt auch nur ein einziger Farbfleck in derjenigen Farbe, die für die Unwirksamkeit bzw. das Verbrauchtsein der Lösung kennzeichnend ist.

Durch die erfindungsgemässen Verfahren wird sichergestellt, dass jederzeit zur Kalibrierung nur brauchbare Null-Kalibrierlösungen verwendet werden. Es ist dann nicht mehr zu befürchten, dass die Unwirksamkeit der Kalibrierlösung übersehen wird. Für die Handhabung im klinischen Betrieb, wo oft höchste Eile bei diagnostischen Messungen geboten ist, ist dies von grosser Bedeutung.

### Patentansprüche

1. Verfahren zum Kalibrieren einer einen Sauerstoff-Messaufnehmer aufweisenden Messanordnung zur Bestimmung des Sauerstoffpartialdrucks in Blut, bei dem zunächst
— der Messaufnehmer mit einer Null-Kalibrierlösung in Berührung gebracht wird, welcher ein Bindemittel zugesetzt ist, das Sauerstoff chemisch zu binden vermag, so dass die Sauerstoff-Konzentration der Kalibrierlösung auf dem Wert Null gehalten wird, und sodann
— die Messanordnung in Betrieb gesetzt und so einreguliert wird, dass sie den Nullwert des Sauerstoffpartialdrucks anzeigt,
dadurch gekennzeichnet, dass
— vor dem ersten Verfahrensschritt die Null-Kalibrierlösung mit einem auf den pH-Wert der Null-Kalibrierlösung ansprechenden Farbindikator versetzt wird, dessen Farbe anzeigt, ob der pH-Wert eine Grösse hat, bei der das Bindevermögen der Null-Kalibrierlösung bzw. des Bindemittels für Sauerstoff noch ausreicht, um dessen Konzentration bzw. Partialdruck auf dem Wert Null zu halten.

2. Verfahren nach Anspruch 1, dadurch gekennzeichnet, dass als Farbindikator Thymolblau verwendet wird.

3. Verfahren nach Anspruch 2, dadurch gekennzeichnet, dass die Null-Kalibrierlösung für jeweils 300 ml Wasser etwa 10 g des Bindemittels und etwa 0,5 bis 10 mg Thymolblau enthält.

4. Anwendung des Verfahrens nach Anspruch 1 zur Kalibrierung von Messaufnehmern für die transcutane Bestimmung des Partialdrucks von Sauerstoff im Blut.

5. Kalibriermittel zur Herstellung einer Null-Kalibrierlösung für das Verfahren nach Anspruch 1, dadurch gekennzeichnet, dass es aus einer Mischung des Sauerstoff-Bindemittels und der Farbindikators in Form von Pulver oder Granulat oder in Tablettenform im erforderlichen Mengenverhältnis besteht und in einem Lösungsmittel, insbesondere in Wasser auflösbar ist.

6. Null-Kalibrierlösung zur Verwendung in einem Verfahren gemäss einem der vorstehenden Verfahrensansprüche, dadurch gekennzeichnet, dass sie aus einer Mischung von Natriumdithionit (Na$_2$S$_2$O$_4$) oder Natriumsulfit (Na$_2$SO$_3$) als Bindemittel in einer Menge von 5 bis 15 g mit dem auf den pH-Wert ansprechenden Farbindikator in Form von Thymolblau in einer Menge von 0,5 bis 10 mg, insbesondere 1 mg, in ca. 300 ml Wasser als Lösungsmittel besteht.

### Claims

1. A process for calibrating a measuring arrangement which is to be used to determine the oxygen partial pressure in blood and which incorporates an oxygen measuring sensor by, first,
— bringing the measuring sensor in contact with a zero-calibrating solution to which has been added a binder which is capable of chemically binding oxygen, so that the oxygen concentration of the calibrating solution is held at a value of zero, and, then,
— setting the measuring arrangement in operation and adjusting it in such a way that it indicates the zero value of the oxygen partial pressure,
which comprises
— admixing, before the first process step, the zero-calibrating solution with a colour indicator which responds to the pH of the zero-calibrating solution and whose colour indicates whether the pH is such that the capacity of the zero-calibrating solution, or of the binder, to bind oxygen is still adequate to keep the oxygen concentration or partial pressure at the value of zero.

2. A process as claimed in claim 1, wherein thymol blue is used as the colour indicator.

3. A process as claimed in claim 2, wherein the zero-calibrating solution contains, per 300 ml of water, about 10 g of the binder and about 0.5 to 10 mg of thymol blue.

4. Application of the process as claimed in claim 1 to calibrating measuring sensors for the transcutaneous determination of the partial pressure of oxygen in the blood.

5. A calibrating agent for preparing a zero-calibrating solution for the process as claimed in claim 1, which comprises a mixture of the oxygen binder and the colour indicator in the form of a powder or granulate or tablets in the required mixing ratio and can be dissolved in a solvent, in particular in water.

6. A zero-calibrating solution for use in a process as claimed in any one of the preceding process claims, which comprises a mixture of sodium dithionite (Na$_2$S$_2$O$_4$) or sodium sulphite (Na$_2$SO$_3$) as binder in an amount of 5 to 15 g, together with the pH-responding colour indicator in the form of thymol blue in an amount of 0.5 to 10 mg, in particular 1 mg, in about 300 ml of water as solvent.

**Revendications**

1. Procédé pour le calibrage d'un dispositif de mesure comportant un capteur de mesure de l'oxygène pour la détermination de la pression partielle de l'oxygène dans le sang, dans lequel:
— on amène tout d'abord le capteur de mesure en contact avec une solution de calibrage du zéro à laquelle on a ajouté un agent de fixation qui permet de fixer chimiquement l'oxygène, de telle sorte que la concentration en oxygène de la solution de calibrage soit maintenue à la valeur nulle
— et qu'ensuite le dispositif de mesure est mis en fonctionnement et réglé de telle manière qu'il affiche la valeur nulle de la pression partielle d'oxygène,
caractérisé par le fait que,
— avant la première étape du procédé la solution de calibrage du zéro reçoit l'adjonction d'un indicateur coloré correspondant à la valeur de pH de la solution de calibrage du zéro et dont la couleur indique si le pH a une valeur pour laquelle l'agent de fixation de la solution de calibrage du zéro ou de l'agent de fixation de l'oxygène suffit encore pour maintenir la concentration ou la pression partielle de ce dernier à la valeur zéro.

2. Procédé selon la revendication 1, caractérisé par le fait que l'on utilise comme indicateur coloré le bleu de Thymol.

3. Procédé selon la revendication 2, caractérisé par le fait que la solution de calibrage du zéro contient pour 300 ml d'eau environ 10 g d'agent de fixation et environ 0,5 à 10 mg de bleu de Thymol.

4. Application du procédé selon la revendication 1, pour le calibrage d'un capteur de mesure pour la détermination transcutanée de la pression partielle de l'oxygène dans le sang.

5. Moyen de calibrage pour la fabrication d'une solution de calibrage du zéro pour le procédé selon la revendication 1, caractérisé par le fait qu'il est constitué par un mélange de l'agent de fixation de l'oxygène et de l'indicateur coloré sous la forme de poudre ou de granulats ou de tablettes et qu'il est soluble dans un solvant, en particulier dans l'eau.

6. Solution de calibrage du zéro pour être utilisée dans un procédé selon l'une quelconque des revendications précédentes, caractérisée par le fait qu'elle est constituée par un mélange de thiosulfate de sodium ($Na_2S_2O_4$) ou de sulfite de sodium ($Na_2SO_3$) en tant qu'agent de fixation en une quantité de 5 à 15 g avec un indicateur coloré correspondant à la valeur du pH sous la forme de bleu de Thymol en une quantité de 0,5 à 10 mg, en particulier 1 mg, dans environ 300 ml d'eau comme agent solvant.